# EUROPEAN PATENT APPLICATION

(11) **EP 2 277 395 A1**
(43) Date of publication of application: **26.01.2011**
(21) Application number: 10170691.9
(22) Date of filing: 23.07.2010
(51) Int. Cl.: A23L 1/30, A23L 1/305

(54) **Composition, method for producing it and use thereof**

(30) Priority: 23.07.2009 NL 1037147
(71) Applicant: Opus Ingredients B.V., 4565 ER Kapellebrug (NL)
(72) Inventor: De Block, Erwin Gerard, 4567 BG, Clinge (NL)
(74) Representative: Van den Heuvel, Henricus Theodorus

(57) **Abstract**

The present invention relates to a composition comprising at least one amino acid selected from the group consisting of valine, isoleucine and leucine, wherein this composition comprises a compound capable of positively influencing the absorption of the amino acid by the gastro-intestinal tract of a human or animal. The invention further relates to a method for producing such a composition comprises the steps of culturing at least one bacterial strain under conditions wherein this bacterial strain is capable of producing the amino acid, and adding the compound capable of positively influencing the absorption of the amino acid by the gastro-intestinal tract of a human or animal. Furthermore, the invention relates to the use of such a composition for the manufacture of a food supplement for improving the food absorption, stimulating growth, improving the immune system, reducing intestinal problems, and/or the general improvement of health.

## Description

### Field of the invention

The present invention relates to a composition comprising at least one amino acid selected from the group consisting of valine, isoleucine and leucine. The invention further relates to a method for producing such a composition and to the use of such a composition.

### Background of the invention

The amino acids valine, isoleucine and leucine belong to the group of the Branched Chain Amino Acids (BCAA's), or branched chained amino acids. BCAA's are important constituents of muscle proteins and are indispensable for the production of additional muscle mass. BCAA's for humans and animals such as pigs and poultry are essential amino acids, meaning that they cannot produce these amino acids themselves. Therefore these amino acids must be absorbed via nutrition or supplements. BCAA's also play an important role in the immune system and the balance between water and hydrogen in the body.

Supplements comprising at least one of these amino acids are consumed by humans to minimize muscle breakdown during exercise and to stimulate muscle building.

Such supplements in animals, in particular animals destined for the meat industry, such as pigs, cattle and poultry, are used for the stimulation of growth. For this in practice a very pure preparation (95-98%) of valine is used. It is known that in essence pure preparations comprising one component are unnatural and therefore when frequently taken are less beneficial for the health.

A further drawback of such compositions is that the amount of valine which is not absorbed in the gastro-intestinal tract and ends up in the environment. This increases the nitrogen discharge while there is in fact a tendency to reduce the nitrogen discharge. Inefficient absorption of valine moreover is a waste of energy and material. A further drawback of these compositions is that they are chemically produced using reagents which are polluting for the environment. Moreover these chemical processes require a lot of energy among others due to heating steps. These drawbacks of the methods of production make these very pure compositions costly.

There is therefore a need for a healthier and preferably cheaper composition comprising at least one amino acid selected from the group consisting of valine, isoleucine and leucine of which the amino acid is better absorbed by the gastro-intestinal tract. There is furthermore a need for a method for producing such a composition, which method uses less polluting reagents and less energy.

A further problem is the addition of antibiotics to animals. In many cases the addition of antibiotics to animal feeds is or will be prohibited. This prohibition is being made because of the development of resistance against such antibiotics by bacteria in the animals, which resistance may be transferred to bacteria which are pathogenic to humans. There is therefore a need for a composition which ends or reduces the use of antibiotics.

The invention therefore provides a composition comprising at least one amino acids selected from the group consisting of valine, isoleucine and leucine, which composition comprises a compound capable of positively influencing the absorption of the amino acid by the gastro-intestinal tract of a human or animal. The compound could relate to influencing the conditions in the gastro-intestinal tract such that the absorption of the amino acid is increased, for example by changing the area of the intestinal pili. The compound will be active during passage of the amino acid through the gastro-intestinal tract whereby the absorption is increased at the suitable moment and a lower amount of the amino acid can be administered. This will also lead to a lower nitrogen discharge in case the composition is used for animals. The ratio of the compound to the amino acid preferably is such that as much amino acid as possible is absorbed and as little as possible of the compound is added.

The mechanism by which the compound positively influences the absorption could also relate to killing of bacteria which negatively influence the absorption of the amino acid or bacteria which themselves absorb the amino acid. In an embodiment of a composition according to the invention the compound therefore has antibacterial action. During the passage of the amino acid preparation through the gastro-intestinal tract the compound will kill bacteria that prevents absorption from the gastro-intestinal tract, thus increasing the absorption and whereby a lower amount of the amino acid must be administered. This could also reduce the total amount of antibacterial compounds which in practice are administered to an animal. Avilamycine, an antibiotic, has proven to increases the absorption capacity of nutrients from the gastro-intestinal tract. A further example of a compound having antibacterial action proven to increase the absorption capacity of nutrients form the gastro-intestinal tract is butyrate (Sakata, 1987; Sakata et al., 1995, Gálfi and Bokor, 1990). However, such a compound is preferably a natural compound, preferably of a vegetable source, preferably a compound known not to have any side effects.

In a preferred embodiment of a composition according to the invention the compound is a Nepetoideae plant or a part thereof or an extract thereof, preferably an oil extract. Genera belong to the family of Nepetoideae are the genus Nepeta, the genus Glechoma, the genus Prunella, the genus Satureja, the genus Clinopodium, the genus Hyssopus, the genus Origanum, the genus Thymus, the genus Lycopus, the genus Mentha, the genus Rosmarinus, the genus Salvia, the genus Ocimum. Of many of these genera of plants it is proven that they have antibacterial action. The compound is of a natural, vegetable source and therefore does not have the described drawbacks of antibiotics. A further advantage of many of such compounds is that they may also serve as a flavoring. Preferably the compound is an *Origanum vulgaris*-plant or part thereof or an extract thereof, preferably an oil extract. *Origanum vulgaris,* is a species belonging to the genus Nepetoideae, and is cultivated for the production of the known spice oregano. The advantage of such an extract is that it is already used for a long time as a flavoring in human and animal nutrition, and during this no harmful effects have been found. Such a compound, in particular the oil-extract, however has additional beneficial features.

Constituents of oregano-oil are carvacrol, thymol, γ-terpinene and p-cymene of which carvacrol and thymol have an antibacterial action "Antimicrobial and Cytotoxic Activities of Origanum Essential Oils" Afroditi Sivropoulou, et al., J. Agric, Food Chem., 1996 44 (5), pp 1202-1205. The constituent carvacrol is able to kill diseased transferring bacteria such as *E. coli* O157:H7 and *Salmonella.* These bacteria are present in food stuffs and may cause serious food infections.

EP0828502 discloses a medicinal composition wherein the anti-inflammatory features of etheric oils selected from the group consisting of *Origanum vulgaris, Thymus vulgaris, menthe piperita, Thymus serpilum, Saturea hortensis, Saturea Montana, Saturea subricata, Carum corticum, Thymus zugis, Ocimum gratisimum, Moranda pungtata, Mosla japanoica* and *Salvia officinalis*, are used for the treatment of medical conditions such as infections caused by fungi and bacteria.

This patent however does not disclose the fact that these etheric oils may improve the absorption of nutrients and may be used as such.

Recently it has been shown that oregano-oil may improve the absorption of nutrients. Ropadiar™ is a commercial preparation of oregano-oil, which preparation has been extensively researched in relation to absorption capacity of nutrients from the gastro-intestinal tract in animals, such as chickens and pigs. In the above cited patent EP0828502 the extraction of the plant for the production of the oil is disclosed. In chickens the ingestion of Ropadiar™ resulted in a better absorption of nutrients and increased egg-production (Case Report of ROPADIAR http://www.pearcesportingservices.co.uk/pss/CaseReportonLayerFarm.pdf). In pigs the ingestion of Ropadiar™ resulted in an increased growth and absorption of nutrients (Praktijkonderzoek Veehouderij rapport 205).

Due to the extensive use thereof it has been shown that oregano-oil, in low concentrations, is a save compound. Furthermore, this compound has been extensively researched in relation to its beneficial effects on the health of humans and animals. There is also experimental evidence that proves that the absorption of valine by animals is positively influenced by an extract of *Oregano vulgaris.* The ratio of the oil-extract of *Oregano vulgaris* to the amino acid is preferably 1:2000-1:20,000. The most optimal ratio depends on the concentration of the active compound in the composition. Of a crude extract usually relatively more is needed to obtain the same effect as for a more purified extract. In an embodiment the compound is the antibacterial compound from oregano-oil, carvacrol. The ratio compound:amino acid can be further increased in this case.

The amino acid preferably is valine. Valine is the most common amino acid administered to animals via supplements. With this amino acid therefore the most experience has been developed.

In a preferred embodiment of a composition according to the invention the composition comprises a biomass, which biomass is formed by the cells of at least one bacterial strain, which bacterial strain under suitable conditions can produce the amino acid. The bacterial strains serve to produce the amino acid prior to the formulation of the composition. Apart from that they may, in case they are and remain viable, also during their residence in the body, in particular in the gastro-intestinal tract, produce the amino acid. They also form a nutritious and vitamin rich source as replenishment to the amino acid whereby the nutrients are also better absorbed by the presence of the compound. Furthermore, bacterial cells are easy to form into powder or pellets which are easily consumed by animals, possibly admixed with their normal feed. With biomass is meant the mass consisting of cells and/or parts of the cells and/or of culture medium and other components usually present in a culture of cells.

The bacterial strain preferably is selected from *Brevibacterium flavum, Escherichia coli, Corynebacterium glutamicum* and *Enterobacteriaceae.* Of these bacterial strains it has been proven that under optimal conditions they are capable of producing high amounts of valine. The skilled person can easily obtain those conditions from literature or may find them experimentally.

The bacterial strain preferably is *Brevibacterium flavum* TV10 or the bacterial strains form a mixture comprising *Brevibacterium flavum* TV10. Of this bacterial strain it has been shown that under optimal conditions it may produced very high amounts of valine.

The ratio of the amino acid to the biomass preferably is within the range of 50:50 to 80:20, and preferably is about 65:35.

The composition preferably has the form of a feed supplement, a sport nutrition supplement, or a pharmaceutical composition for the treatment or prevention of a condition. The feed supplement may be used for the same conditions as the present pure preparation of about 97%. Thus for the improvement of the growth of animals such as pigs or poultry. The composition may also be used by humans for the building of muscle mass, to be used prior to or after exercise. The supplement may optionally comprise food constituents such as a carbohydrate source, a texture improver for improving the palatability etc.

The composition preferably is in powder form. Such a form is easy to produce and to mix with other food ingredients. The powder may prior to use optionally form a suspension or solution and may be administered as such.

The invention also provides a method for producing a composition as described above, which method comprises, culturing at least one bacterial strain under conditions wherein this bacterial strain is capable of producing the amino acid, and adding a compound capable to positively influence the absorption of the amino acid by the gastro-intestinal tract of a human or animal, for example an oil-extract of *Origanum vulgaris.*

Preferably step b) is performed after step a) in case the compound has antibacterial action. An advantage of such a method is that it does not make use of environmentally polluting reagents. Also such a method is energy efficient because the temperature only needs to be increased for growing the bacterial strain. After the culturing only the compound needs to be added. The compound may be a commercially available compound. The conditions for growing the bacterial strain(s) are known and/or easy to investigate by a person skilled in the field. As a substrate for the bacterial strain usually glucose is used. When the compound has antibacterial action the bacterial cells may be killed by the compound and the composition is also protected against contamination by other pathogenic bacteria.

The method preferably also comprises the step of concentrating the culture and treating the concentrate such that it assumes a easily ingestible form, such as a powder or pellet form. The liquid culture may be concentrated by means of centrifugation or filtration. The concentrate can be extruded for producing the correct form.

The bacterial strain preferably is selected form *Brevibacterium flavum*, *Escherichia coli, Corynebacterium glutamicum* and *Enterobacteriaceae.* The bacterial strain preferably is *Brevibacterium flavum* TV10 or the bacterial strains preferably form a mixture comprising *Brevibacterium flavum* TV10.

The invention further provides the use of a composition according to the invention in the manufacture of a food supplement for the improvement of the food absorption, the improvement of the growth, the improvement of the immune system, reducing intestinal problems and/or a general improvement of the health. The formulation of the supplement may be adjusted specifically for humans or animals.

## Claims

1. Composition comprising at least one amino acid selected form the group consisting of valine, isoleucine, and leucine, **characterized in that** the composition comprises a compound capable of positively influencing the absorption of the amino acid by the gastro-intestinal tract of a human or animal, wherein the compound preferably has antibacterial action.

2. Composition according to claims 1, **characterized in that** the compound is a Nepetoideae-plant or a part thereof or an extract thereof, preferably an oil-extract, wherein the Nepetoideae-plant preferably is selected from *Origanum vulgaris.*

3. Composition according to any of the previous claims, **characterized in that** the compound is carvacrol.

4. Composition according to any of the previous claims, **characterized in that** the ratio of the compound to the amino acid is 1:2000-1:20,000.

5. Composition according to any of the previous claims, **characterized in that** the composition comprises a biomass, which biomass is formed by the cells of at least one bacterial strain, which bacterial strain under suitable conditions can produced the amino acid, wherein at least one bacterial strain preferably is selected from *Brevibacterium flavum, Escherichia coli, Corynebacterium glutamicum* or *Enterobacteriaceae*, and most preferably comprises a selection from *Brevibacterium flavum* TV 10.

6. Composition according to any of the previous claims, **characterized in that** ratio of the amino acid to the biomass is within the range of 50:50 to 80:20, and preferably is about 65:35.

7. Composition according to any of the previous claims, **characterized in that** it has the form of an animal feed supplement, a sport nutritional supplement, or a pharmaceutical composition for the treatment or the prevention of a condition.

8. Method for producing a composition according to any of the previous claims, **characterized in that** it comprises the steps of,
a) culturing at least one bacterial strain under conditions wherein this bacterial strain is capable of producing the amino acid, and
b) adding the compound capable of positively influencing the absorption of the amino acid by the gastro-intestinal tract of a human or animal.

9. Method according to claim 8, **characterized in that** the compound is added directly to the culture.

10. Method according to claim 8 or 9, **characterized in that** it further comprises the step of,
a) concentrating the culture
b) treating the concentrate such that it assumes an easily ingestible form, such as a powder or pellet form.

11. Method according to claim 8, 9 or 10, **characterized in that** at least one bacterial strain is selected from *Brevibacterium flavum, Escherichia coli, Corynebacterium glutamicum* or *Enterobacteriaceae*, wherein the bacterial strain most preferably comprises a selection from *Brevibacterium flavum* TV10.

12. Use of a composition according to any of the claims 1-7 for the manufacture of a food supplement for
a) improving the food absorption;
b) stimulating growth;
c) improving the immune system;
d) reducing intestinal problems, and/or
e) the general improvement of health.
